(19) Europäisches Patentamt
European Patent Office
Office européen des brevets

(11) EP 2 885 305 B1

(12) FASCICULE DE BREVET EUROPEEN

(45) Date de publication et mention de la délivrance du brevet:
**01.03.2017 Bulletin 2017/09**

(21) Numéro de dépôt: **13758931.3**

(22) Date de dépôt: **05.08.2013**

(51) Int Cl.:
***C07D 493/04*** *(2006.01)*

(86) Numéro de dépôt international:
**PCT/FR2013/051881**

(87) Numéro de publication internationale:
**WO 2014/023902 (13.02.2014 Gazette 2014/07)**

(54) **PROCEDE DE PREPARATION DE DIALKYLOXYDIANHYDROHEXITOLS PAR ETHERIFICATION DE DIANHYDROHEXITOLS AVEC UN ALCOOL LEGER, EN PRESENCE D'UN CATALYSEUR ACIDE**

VERFAHREN ZUR HERSTELLUNG VON DIALKYLOXYDIANHYROHEXITOL MITTELS VERETHERUNG VON DIANHYDROHEXITOL MITHILFE EINES LEICHTEN ALKOHOLS UNTER VERWENDUNG EINES SAUREN KATALYSATORS

METHOD FOR PREPARING DIALKYLOXYDIANHYROHEXITOL BY ETHERIFICATION OF DIANHYDROHEXITOL USING A LIGHT ALCOHOL, IN THE PRESENCE OF AN ACIDIC CATALYST

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.08.2012 FR 1257621**

(43) Date de publication de la demande:
**24.06.2015 Bulletin 2015/26**

(73) Titulaires:
- **Roquette Frères**
  **62136 Lestrem (FR)**
- **Centre National de la Recherche Scientifique (C.N.R.S.)**
  **75794 Paris Cedex 16 (FR)**
- **Université Claude Bernard Lyon 1**
  **69622 Villeurbanne, Cedex (FR)**

(72) Inventeurs:
- **IBERT, Mathias**
  **F-59930 La Chapelle d'Armentieres (FR)**
- **ESSAYEM, Nadine**
  **F-38540 Saint Just Chaleyssin (FR)**
- **FECHE, Cyril**
  **F-01150 Leyment (FR)**
- **PERRARD, Alain**
  **F-69110 Sainte-Foy-les-Lyon (FR)**

(74) Mandataire: **Cabinet Plasseraud**
**235 Cours Lafayette**
**69006 Lyon (FR)**

(56) Documents cités:
**WO-A2-2009/120703**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 885 305 B1

## Description

### Domaine de l'invention

Le domaine de l'invention est celui de la O-alkylation de dianhydrohexitols.

En particulier, la présente invention est relative à la préparation de dérivés éthers de 1,4:3,6-dianhydrohexitol tels que l'isosorbide, l'isoidide ou l'isomannide.

Plus précisément, l'invention vise un nouveau procédé industriel d'éthérification de ces dianhydrohexitols, à l'aide d'alcools légers, du type méthanol, éthanol, par catalyse acide ou catalyse bifonctionnelle acide-métal, de préférence en phase gazeuse.

### Arrière-plan technologique et art antérieur

Les 1,4:3,6-dianhydrohexitols connus sont notamment : l'isosorbide, l'isomannide et l'isoidide de formule :

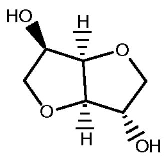

**Isosorbide**

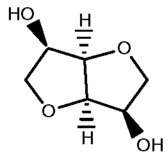

**Isomannide**

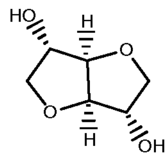

**Isoidide**

On connaît également des dérivés de l'isosorbide, l'isomannide et l'isoidide dans lesquels les fonctions réactives -OH sont remplacées par des fonctions réactives amine, acide ou éther.

Le diméthylisosorbide (DMI)

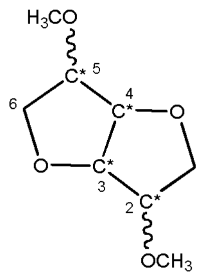

est un exemple de dérivé éther d'isosorbide. Le DMI est un solvant préconisé dans les compositions pharmaceutiques et cosmétiques telles que les compositions autobronzantes, bucco-dentaires ou antiacnéiques, les crèmes de soins, les pommades et les lotions. Le DMI est aussi un agent de contrôle de la viscosité. Il peut être utilisé comme fluxant dans les bitumes.

L'utilisation du DMI dans de nombreux domaines autres que l'industrie pharmaceutique et cosmétique est notamment décrite dans les demandes internationales WO 2006/120342 et WO 2006/120343 de la demanderesse.

Le DMI est classiquement préparé par méthylation de l'isosorbide avec un agent de méthylation tel que le sulfate de diméthyle ou le chlorure de méthyle, en présence d'un agent alcalin tel que la soude. Le chlorure de méthyle est un agent de méthylation particulièrement intéressant pour des raisons économiques. Il est en effet disponible sur le marché en grande quantité et à un coût inférieur à celui des équivalents bromés et iodés. La demande de brevet EP 0 092 998 décrit ainsi la méthylation d'isosorbide par du chlorure de méthyle (MeCl), en présence d'hydroxyde de sodium ou de potassium. La réaction décrite est mise en oeuvre dans une dispersion eau/solvant organique aprotique (DMSO ou toluène) avec barbotage de l'agent de méthylation gazeux.

Bien qu'elle offre de hauts rendements en DMI (90-95%), cette méthylation dans un milieu aqueux pose toutefois les problèmes suivants :

(i) Hydrolyse de l'agent de méthylation. Cette réaction secondaire indésirable réduit en effet le rapport [MeCl fixé/MeCl introduit], appelé ci-après taux de fixation de MeCl, et aboutit à la formation d'importantes quantités de sels, généralement de chlorure de sodium ou chlorure de potassium, qui doivent être retraitées à l'issue du procédé.

(ii) Réactif gazeux délicat à mettre en oeuvre

(iii) Toxicité du réactif chlorure de méthyle
(iv) Toxicité du solvant (DMSO ou toluène).

L'emploi de dialkyl-carbonate (diméthyl ou diéthyl) pour obtenir du DMI à partir d'isosorbide, ne comporte pas ces inconvénients (i)(ii)(iii)(iv). Cet agent de méthylation est utilisé à la fois comme réactif et solvant "vert". Il est utilisé en présence d'un catalyseur basique. La réaction a lieu à des températures et pressions élevées et utilise un seul groupe méthyle du diméthyle carbonate, ce qui pénalise l'économie du procédé (US 4,770,871 ; WO 2009/120703). En particulier, cette dernière demande internationale de brevet WO 2009/120703 décrit un procédé pour éthérifier les sucres dianhydrohexitols en présence d'un agent O-alkylant qui est un dialkyl-carbonate.
Il existe donc un besoin d'un procédé propre de synthèse du DMI, et plus généralement de di, sans génération de sels en particulier, avec un agent de méthylation peu coûteux et efficace (sans perte de carbone).
Force est de constater que l'art antérieur ne satisfait pas à ce besoin.

## Objectifs

Dans ce contexte, la présente invention vise à satisfaire à au moins l'un des objectifs ci-dessous énoncés.
L'un des objectifs essentiels de la présente invention est de fournir un nouveau procédé perfectionné de préparation d'une composition à base de dialkyloxydianhydrohexitols par éthérification de dianhydrohexitols avec au moins un agent d'alkylation.
Un autre objectif essentiel de l'invention est de fournir un nouveau procédé perfectionné, simple et économique, de préparation d'une composition à base de dialkyloxydianhydrohexitols par éthérification de dianhydrohexitols avec au moins un agent d'alkylation.
Un autre objectif essentiel de l'invention est de fournir un nouveau procédé perfectionné de préparation d'une composition à base de dialkyloxydianhydrohexitols par éthérification de dianhydrohexitols avec au moins un agent d'alkylation, ledit procédé ne générant pas de sous-produit embarrassant.
Un autre objectif essentiel de l'invention est de fournir un nouveau procédé perfectionné de préparation d'une composition à base de dialkyloxydianhydrohexitols par éthérification de dianhydrohexitols avec au moins un agent d'alkylation peu coûteux.
Un autre objectif essentiel de l'invention est de fournir un nouveau procédé perfectionné de préparation d'une composition à base de dialkyloxydianhydrohexitols par éthérification de dianhydrohexitols avec au moins un agent d'alkylation non toxique, non dangereux et écocompatible.
Un autre objectif essentiel de l'invention est de fournir un nouveau procédé perfectionné de préparation d'une composition à base de dialkyloxydianhydrohexitols par éthérification de dianhydrohexitols avec au moins un agent d'alkylation, ledit procédé étant aisément industrialisable et pouvant à ce titre être, par exemple, mis en oeuvre en continu.
Un autre objectif essentiel de l'invention est de fournir un nouveau procédé perfectionné de préparation d'une composition à base de dialkyloxydianhydrohexitols par éthérification de dianhydrohexitols avec au moins un agent d'alkylation, ledit procédé bénéficiant d'une bonne thermodynamique, et donc d'une bonne cinétique réactionnelle.
Un autre objectif essentiel de l'invention est de fournir un nouveau procédé perfectionné de préparation d'une composition à base de dialkyloxydianhydrohexitols par éthérification de dianhydrohexitols avec au moins un agent d'alkylation, avec de bons rendements et de bonnes sélectivités en dialkyloxydianhydrohexitols.
Un autre objectif essentiel de l'invention est de fournir un nouveau procédé perfectionné de préparation d'une composition à base de diméthylisosorbide par éthérification d'isosorbide avec du méthanol, ledit procédé satisfaisant à au moins l'un des objectifs susvisés.

## Brève description de l'invention

Ces objectifs, parmi d'autres, sont atteints par la présente invention qui concerne tout d'abord un procédé de préparation d'une composition à base de dialkyloxydianhydrohexitols par éthérification de dianhydrohexitols avec au moins un agent d'alkylation, en présence d'un catalyseur solide, de préférence un catalyseur présentant des propriétés d'acides de Lewis ou de BrØnsted, l'agent d'éthérification étant choisi dans le groupe comprenant, et mieux encore constitué par :

- les alcools, de préférence les alcools aliphatiques linéaires ou ramifiés, et, plus préférentiellement encore les alcools en C1-C20, mieux encore le méthanol, l'éthanol, l'isopropanol ou le tertio-butanol; le méthanol étant particulièrement préféré ;
- et leurs mélanges.

Ce nouveau procédé performant est parfaitement adapté à une exploitation industrielle. Il permet la synthèse de méthyl-isosorbide éthers par réaction de l'isosorbide avec le méthanol (ou éthanol), en présence d'un catalyseur solide acide.

Le procédé selon l'invention est "propre", il contourne l'emploi d'agent de méthylation tel que le sulfate de diméthyle ou de chlorure de méthyle qui génère des quantités stoechiométriques de sels. Il évite aussi l'emploi de dialkyl-carbonate, agent de méthylation plus coûteux dont un seul des deux groupes méthyles participe à l'obtention des éthers mixtes de l'isosorbide.

*Définitions*

Dans le présent exposé, tout singulier désigne indifféremment un singulier et un pluriel et réciproquement, sauf indication contraire expresse.

Les définitions suivantes sont données à titre d'exemples pour l'interprétation du présent exposé.

- Par "*dialkyloxydianhydrohexitol*" on entend un dérivé de 1,4:3,6-dianhydrohexitol de formule III :

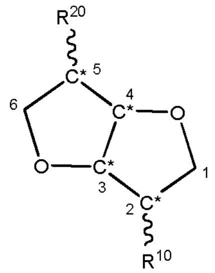

(III)

dans laquelle :

$R^{10}$, $R^{20}$ est un radical $-OR^{30}$, les radicaux $R^{30}$ étant identiques ou différents et correspondant chacun à un alkyle, de préférence un alkyle aliphatique linéaire ou ramifié et, plus préférentiellement encore, un alkyle en C1-C20, mieux encore le méthyle, l'éthyle, l'isopropyle ou le tertio-butyle; le méthyle étant particulièrement préféré, ce qui correspond au DiMéthylIsosorbide (DMI) comme composé de formule (III).

- Par "*O-alkylation*" on entend greffage d'un alkyle par liaison éther sur un produit.

- Par "*compris entre x et y*", on entend un ou des intervalles de valeurs dont les bornes sont fermées : [x,y].

- Par "*catalyseurs solide*" on entend un composé chimique solide qui constitue une phase distincte de la phase réactionnelle capable d'exercer sur l'évolution d'une transformation thermodynamiquement possible, un effet accélérateur et un effet d'orientation et se retrouvant inaltéré en fin de réaction et ne pouvant modifier l'équilibre thermodynamique («Catalyse de contact» - J.P. Le Page-Editions Technip-1978, pages 1, 2).

- Par "*catalyseur solide supporté.*" on entend un catalyseur solide constitué d'un support inerte de grande surface spécifique sur lequel est dispersé un composé chimique catalytiquement actif («Catalyse de contact» - J.P. Le Page-Editions Technip-1978, page 133).

- Par "*Conversion*", notamment "*conversion de l'isosorbide*" (notée également « Conv isosorbide »), on entend :

Conv isosorbide (%) = 100*(nombre initial de mole(s) d'isosorbide – nombre final de mole(s) d'isosorbide) / nombre initial de mole(s) d'isosorbide.

- Par "*sélectivité*" (notée également Sé), on entend :

Sé produit i (% mol)= (100*nb moles produit i)/ (somme nb moles produits i),

i désignant les dérivés de l'isosorbide : DMI ou MMI A OU MMI B.

Dans la présente demande, les sélectivités sont ainsi calculées par normalisation à 100% molaire sur les dérivés de l'isosorbide.

**Description détaillée de l'invention**

*Préférences*

- *Mode préféré de mise en oeuvre: phase gazeuse*

Dans ce mode préféré de mise en oeuvre, l'éthérification est au moins en partie effectuée en phase gazeuse.
La réalisation de la réaction en phase gazeuse permet notamment l'obtention de produits de réaction non colorés.

- *Catalyseur*

De préférence, le catalyseur est choisi dans le groupe comprenant et, mieux encore, constitué par :

1. les sels des hétéropolyacides ou polyoxométallates de formule générale :

$$H_kX_jM_mO_n,\ yH_2O \qquad (I)$$

dans laquelle,

- X représente un hétéroatome choisi dans le groupe constitué par les éléments suivants : P, Si, Ge, B et As,
- M représente un élément métallique périphérique choisi dans le groupe constitué par W, Mo et V,
- j est le nombre d'hétéroatomes et représente 1 ou 2,
- k est le nombre d'atomes d'hydrogène et est compris entre 0,5 et 10,
- m est le nombre d'atomes métalliques périphériques W, Mo, V et est compris entre 1 et 18,
- n est le nombre d'atomes d'oxygène et est compris entre 2 et 62,
- y est le nombre de molécules d'eau d'hydratation et est compris entre 0 et 40, de préférence entre 6 et 30,

et leurs mélanges;
2. les sels de métaux alcalins $Cs^+$, $K^+$, $Rb^+$ et les sels d'ammonium ($NH_4^+$), ces derniers étant préférés;
et leurs mélanges;
3. les catalyseurs acides à base d'oxyde de zirconium modifié par des oxoanions de type sulfate ou tungstate d'appellation ZrS ou ZrW pouvant contenir des métaux de transition tels Fe, Mn et leurs mélanges;
4. les zéolithes, de préférence choisies dans le groupe comprenant et, mieux encore, constitué par : H-Béta ; H-ZSM-5 ; MCM-22 ; H-USY ; et leurs mélanges ;
5. les argiles acides de type montmorillonites, les phosphates tels que le phosphate de Nb ou de zirconium, les charbons fonctionnalisés, notamment les charbons fonctionnalisés par des groupes sulfoniques;
6. et leurs mélanges.

De préférence, pour le catalyseur, les sels des hétéropolyacides (polyoxométallates) de formule générale (I) sont choisis dans le groupe comprenant et, mieux encore, constitué par : $H_3PW_{12}O_{40}$,$21H_2O$ ; $H_4SiW_{12}O_{40}$,$24H_2O$ ; $H_6P_2W_{18}O_{62}$,$24H_2O$ ; $H_5BW_{12}O_{40}$,$30H_2O$ ; $H_5PW_{10}V_2O_{40}$,$yH_2O$ ; $H_3PMo_{12}O_{40}$,$28H_2O$ ; $H_4SiMo_{12}O_{40}$,$13H_2O$ ; $H_3PMo_6V_6O_{40}$,$yH_2O$ et $H_5PMo_{10}V_2O_{40}$, $yH_2O$ ; et leurs mélanges.
Avantageusement, le catalyseur solide est un catalyseur supporté.

- *Catalyseur bifonctionnel*

Suivant une possibilité intéressante offerte par l'invention, le catalyseur est un catalyseur bifonctionnel métal-acide, c'est-à-dire que :

(i) le catalyseur comprend un métal noble (de préférence choisi dans le groupe comprenant et, mieux encore, constitué par l'or, le platine, le palladium, le ruthénium éventuellement modifiés par l'ajout de rhénium, d'osmium et d'iridium, de titane, de zirconium, de tantale, leurs mélanges ou leurs alliages),
(ii) et l'éthérification est au moins en partie effectuée sous flux d'hydrogène.

L'utilisation d'un catalyseur bifonctionnel métal-acide et l'ajout d'hydrogène au flux des réactifs, en particulier lorsque ceux-ci sont gazeux, permettent de stabiliser l'activité catalytique. On lutte ainsi contre la perte d'activité du catalyseur due par exemple à un empoisonnement des sites acides par forte adsorption d'oligomères. Le catalyseur bifonctionnel métal-acide permet l'hydrogénation in situ des précurseurs de ces oligomères.
Suivant une possibilité, le catalyseur solide est choisi parmi ceux ayant une chaleur différentielle d'adsorption d'ammoniac (en kJ/mole) supérieure ou égale à 100, de préférence à 120 ou, mieux encore comprise entre 120 et 200.
Par "chaleur différentielle d'adsorption d'ammoniac Qdiff', on entend, par exemple, la quantité de chaleur dégagée dQ par l'adsorption d'une quantité infiniment petite d'ammoniac gazeux dn à température constante sur le catalyseur initialement sous vide Qdiff= dQ/dn exprimée en kJ/mole selon « Les techniques physiques d'étude des catalyseurs » Edition Technip - Editeurs, B.Imelik, J.C.Védrine, 1988, telle que définie ci-après dans les exemples.

- *Régénération catalyseur*

Un autre moyen avantageux pour s'opposer à la perte d'activité du catalyseur est de faire en sorte que le procédé comprenne une étape de régénération du catalyseur solide, de préférence par un traitement sous $O_2$ à haute température.
Par "*haute température*" de régénération, on vise par exemple des températures (°C) comprises entre, dans un ordre croissant de préférence 400 et 600°C ou mieux encore 450 et 500°C.
Des cycles de régénération du catalyseur usagé par un traitement sous oxygène à haute température permettent de régénérer son activité et de lui conférer une résistance à l'empoisonnement.

- *Réactifs*

Suivant une modalité préférée de l'invention :

- les dianhydrohexitols comprennent un dérivé de 1,4:3,6-dianhydrohexitol de formule II :

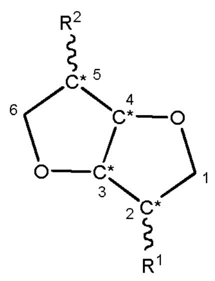

(II)

dans laquelle :

$R^1$, $R^2$ est un radical $-OR^3$, les radicaux $R^3$ étant identiques ou différents et correspondant chacun à H ou un alkyle ;

- et les dialkyloxydianhydrohexitols comprennent un dérivé de 1,4:3,6-dianhydrohexitol de formule III :

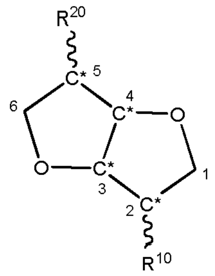

(III)

dans laquelle :

$R^{10}$, $R^{20}$ est un radical $-OR^{30}$, les radicaux $R^{30}$ étant identiques ou différents et correspondant chacun à un alkyle, de préférence un alkyle aliphatique linéaire ou ramifié, et, plus préférentiellement encore, un alkyle en C1-C20, mieux encore le méthyle, l'éthyle, l'isopropyle ou le tertio-butyle; le méthyle étant particulièrement préféré, ce qui correspond au DiMéthylIsosorbide (DMI) comme composé de formule (III).

L'agent d'éthérification est choisi dans le groupe comprenant ou, mieux encore, constitué par :

◦ les alcools, de préférence les alcools aliphatiques linéaires ou ramifiés, plus préférentiellement encore les alcools en C1-C20, mieux encore le méthanol, l'éthanol, l'isopropanol ou le tertio-butanol ; le méthanol étant particulièrement préféré ;

o et leurs mélanges.

▪ *Données quantitatives*

Suivant une autre caractéristique remarquable de l'invention, le rapport molaire [agent d'alkylation / dianhydrohexitol] est inférieur ou égal à, dans un ordre croissant de préférence : 30 ; 25 ; 20; 10; 5 ; 4; 3 ; 2 ou mieux encore compris entre 2 et 20.

▪ *Méthodologie*

De préférence, le procédé est mis en oeuvre selon un mode continu ou semi-continu. La réaction est avantageusement mise en oeuvre dans un réacteur continu et en phase gazeuse à haute température. Par "*haute température*" de réaction, on vise par exemple des températures (°C) supérieures ou égales à, dans un ordre croissant, de préférence 160-300 et mieux encore comprise entre 180-240.

La mise en oeuvre dans un réacteur continu présente l'avantage de donner un produit de réaction non coloré contrairement à la mise en oeuvre en réacteur batch en phase liquide, caractérisée par des temps de contact plus longs, favorables à la formation de produits secondaires généralement colorés, vraisemblablement des oligomères des dianhydrohexitols (*e.g.* isosorbide).

Sur le plan thermique, il est avantageux que :

1. dans une première étape, on vaporise le(s) dianhydrohexitol(s) à une température de T1 (en °C) supérieure ou égale à 170, de préférence à 180; T1 étant plus préférentiellement encore comprise entre 190 et 300 ;
2. et dans une deuxième étape, on effectue l'éthérification avec l'agent d'alkylation à une température T2 (en °C) supérieure ou égale à T1, de préférence supérieure ou égale à 180 ; T2 étant plus préférentiellement encore comprise entre 200 et 300.

Suivant une caractéristique remarquable de l'invention, le(s) dianhydrohexitol(s) de départ est fondu, en solution et/ou provient directement de la synthèse de dianhydrohexitol(s) à partir d'hexitol(s). Avantageusement, le(s) dianhydrohexitol(s) de départ provient directement d'une étape de purification réalisée lors de la synthèse de dianhydrohexitol(s) à partir d'hexitol(s), notamment d'une étape de distillation. La déshydratation de l'hexitol peut être catalysée par le catalyseur d'éthérification dans une étape unique intégrant la déshydratation de l'hexitol en dianhydrohexitol et l'éthérification du dianhydrohexitol.

Par ailleurs, étant donné que la réaction d'éthérification des dianhydrohexitols (e.g. isosorbide) est une réaction successive conduisant à l'obtention de monoalkyléthers (e.g. monométhyléthers ou monoéthyléthers) A et B et de

dialkyléthers (*e.g.* diméthyléther ou diéthyléthers d'isosorbide), il est apparu intéressant, selon une modalité particulière de l'invention, de mettre en place une boucle de recirculation des produits de réaction de façon à favoriser l'obtention des produits finaux, à savoir les dialkyloxydianhydrohexitols (*e.g.* le diméthylisosorbide).

- *Applications*

Le procédé selon l'invention est un procédé industriel utilisable par des producteurs d'hexitols tels que le sorbitol ou d'anhydrohexitol comme l'isosorbide.

Ce procédé conduit à une composition à base de dialkyloxydianhydrohexitols (*e.g.* éthers de l'isosorbide comme le diméthyléther ou le diéthyléther). Ces produits trouvent des applications notamment en tant que fluxant pour bitume, en tant que solvant, ou dans des compositions pharmaceutiques ou cosmétiques.

D'autres détails de l'invention apparaîtront plus clairement au vu des exemples donnés ci-dessous à titre indicatif.

## EXEMPLES

### 1. Dispositif

1.1 Phase liquide

Le réacteur utilisé est un autoclave équipé d'un agitateur magnétique. Les réactifs liquides sont introduits, l'alcool puis l'isosorbide, enfin le catalyseur solide. L'autoclave est inerté sous 20 bars d'argon. Il est porté à la température de réaction au moyen de résistances électriques.

1.2 Phase gazeuse - Mode continu

Ce dispositif, représenté sur la figure 1 annexée, comprend :

1. un four de vaporisation
2. un four réactionnel dans le prolongement du four de vaporisation 1
3. une arrivée (conduit et pompe) du mélange de réactifs
4. une alimentation en azote pour inerter les enceintes des fours de vaporisation 1 et réactionnel 2
5. un serpentin dans le four de vaporisation 1
6. une chambre réactionnelle disposée dans le four réactionnel 2 et comprenant le catalyseur 6
7. un condenseur en aval du four réactionnel 2
8. un bain réfrigérant associé au condenseur 7
9. et un conduit de sortie de l'isosorbide O-méthylé.

### 2. Réactifs

L'isosorbide (ROQUETTE FRERES) est conservé au réfrigérateur sous atmosphère inerte. Le méthanol et l'éthanol proviennent du fournisseur Aldrich.

### 3. Techniques de caractérisation utilisées

L'analyse des produits de réaction est réalisée par chromatographie en phase gazeuse équipée d'une colonne DB1 30m $\times$ 0,32mm, après silylation au moyen de BSTFA (N,O-bis-(triméthylsilyltrifluoroacetamide)).

### Exemple 1 : O-méthylation de l'isosorbide par le méthanol en présence d'un sel acide de potassium de l'acide 12-tungstophosphorique : $K_2HPW_{12}O_{40}$ en réacteur batch, phase liquide.

Les quantités suivantes sont introduites dans l'autoclave : catalyseur = 2g, isosorbide = 36g, rapport molaire MeOH/isosorbide = 5. Le catalyseur est le sel acide de césium de l'acide 12-tungstophosphorique : $K_2HPW_{12}O_{40}$.

Au démarrage de la réaction, le ciel de l'autoclave est constitué de 20 bars d'Ar.

Le mélange réactionnel est porté à deux températures différentes : 180°C ou 200°C. La durée de la réaction est de 6h.

En fin de réaction, le milieu réactionnel est refroidi au moyen d'un bain de glace.

Les produits liquides de réaction sont séparés du milieu réactionnel et analysés par chromatographie gazeuse.

La conversion de l'isosorbide et les sélectivités sont calculées en % molaires (*normalisation à 100% mol des conversions et des sélectivités sur les dérivés de l'isosorbide*).

Les résultats sont présentés dans le tableau 1.

**Tableau 1 :** Conversion et sélectivités de la réaction d'éthérification de l'isosorbide par le méthanol catalysée par $K_2HPW_{12}O_{40}$ en réacteur batch, en phase liquide. Influence de la température de réaction.

| T(°C) | Conversion Isosorbide (%) | Sélectivités (% molaire) | | |
|---|---|---|---|---|
| | | DMI | MMI B | MMI A |
| 180 | 26 | 7 | 46 | 35 |
| 200 | 34 | 9 | 35 | 37 |

La formation des composés mono-méthylés A et B (MMI A et MMI B) et du diméthylisosorbide (DMI) est observée à 180°C et 200°C avec le catalyseur $K_2HPW_{12}O_{40}$. Cependant, la sélectivité pour le DMI est faible, en particulier inférieure à 10%.

En phase liquide, les milieux réactionnels obtenus aux deux températures 180 et 200°C sont fortement colorés.

La coloration s'intensifie avec l'augmentation de la température de réaction.

**Exemple 2 : O-méthylation de l'isosorbide par le méthanol catalysée par un sel acide de potassium de l'acide 12-tungstophosphorique : $K_2HPW_{12}O_{40}$ en réacteur continu en phase gazeuse**

La réaction est mise en oeuvre dans le dispositif de la figure 1.

Le protocole expérimental suivant a été adopté:

- Prélèvement du condensat au bout d'une heure (1h) et de quatre heures de réaction (4h),
- Arrêt du pompage du mélange réactionnel au bout de quatre heures de réaction.

Les conditions expérimentales ont été les suivantes :

Catalyseur : $K_2HPW_{12}O_{40}$, $m_{cata}$ = 2g
$D_{liq}$ = 0,06 $mL.min^{-1}$
$D_{N2}$ = 8 $mL.min^{-1}$
$P_{méthanol}$ = 600 torr
$P_{isosorbide}$ = 28 torr
$T_{vaporisation}$ = 225°C
$T_{réaction}$ = 225°C
Rapport molaire méthanol/isosorbide = 20
$ppH_{iso}$ ($h^{-1}$) = Débit massique isosorbide ($g.h^{-1}$)/masse de catalyseur (g) = 0,26 $h^{-1}$

Les résultats obtenus sont présentés dans le tableau 2.

**Tableau 2 :** O-méthylation de l'isosorbide par le méthanol catalysée par $K_2HPW_{12}O_{40}$ en réacteur continu en phase gazeuse

| | $K_2HPW_{12}O_{40}$ | |
|---|---|---|
| Prélèvement (h de réaction) | 1h | 4h |
| Conv isosorbide (%) | 57 | 32 |
| Sé DMI (mol %) | 62 | 60 |
| Sé MMI B (mol %) | 30 | 22 |
| Sé MMI A (mol%) | 8 | 18 |
| MMI : Monométhyl d'isosorbide | | |

Après une heure de réaction, la conversion d'isosorbide (Conv isosorbide) est de 57% avec une formation majoritaire de DMI. La sélectivité en DMI (Sé DMI) est de 62%. Entre 1h et 4h, l'activité se stabilise à un taux de

conversion d'isosorbide d'environ 32%.

Une très bonne conversion d'isosorbide en DMI est donc obtenue en phase gaz par comparaison à celle obtenue en phase liquide (exemple 1). Par ailleurs, aucune coloration du milieu réactionnel n'a eu lieu au cours de la réaction en phase gazeuse. Ceci témoigne notamment d'une absence de dégradation des produits de réaction malgré une température de réaction élevée.

**Exemple 3 : O-méthylation de l'isosorbide par le méthanol catalysée par des catalyseurs solides acides zéolithiques en réacteur continu en phase gazeuse à haute température**

La réaction est mise en oeuvre dans le même dispositif que celui de l'exemple 2.

Le protocole expérimental suivant a été adopté :

- prélèvement du condensat au bout d'une heure et de 4h de réaction
- arrêt du pompage du mélange réactionnel au bout de quatre heures de réaction avec maintien du débit d'azote pendant 30 minutes.

Les conditions expérimentales étaient les suivantes :

$m_{cata}$ = 2g
$D_{liq}$ = 0,06 $mL.min^{-1}$
$D_{N2}$ = 8 $mL.min^{-1}$
$P_{méthanol}$ = 600 torr
$P_{isosorbide}$ = 28 torr
$T_{vaporisation}$ = 225°C
$T_{réaction}$ = 205°C
Rapport molaire méthanol/isosorbide = 20
$ppH_{iso}$ ($h^{-1}$) = Débit massique isosorbide ($g.h^{-1}$)/masse de catalyseur (g) = 0,26 $h^{-1}$

Les résultats obtenus après 1h et 4h de réaction sont présentés dans les tableaux 3 et 4 respectivement.

**Tableau 3 :** O-méthylation de l'isosorbide par le méthanol catalysée par des catalyseurs solides acides zéolithiques en réacteur continu en phase gazeuse. Résultats après 1h de réaction.

| Catalyseurs | Conversion Isosorbide (%) | Sélectivités (% molaire) | | |
|---|---|---|---|---|
| | | DMI | MMI B | MMI A |
| Beta | 78 | 34 | 54 | 12 |
| ZSM5 | 76 | 33 | 54 | 13 |
| MCM-22 | 51 | 9 | 73 | 18 |
| USY | 70 | 35 | 47 | 18 |

Les zéolithes catalysent l'éthérification de l'isosorbide par la MeOH. La proportion de diméthyléther formée par déshydratation intramoléculaire dépend de la zéolithe.

**Tableau 4 :** O-méthylation de l'isosorbide par le méthanol catalysée par des catalyseurs solides acides zéolithiques en réacteur continu en phase gazeuse. Résultats après 4h de réaction.

| Catalyseurs | Conversion Isosorbide (%) | Sélectivités (% molaire) | | |
|---|---|---|---|---|
| | | DMI | MMI B | MMI A |
| Beta | 42 | 16 | 51 | 33 |
| ZSM5 | 31 | 14 | 47 | 39 |
| MCM-22 | 8 | 2 | 50 | 48 |
| USY | 35 | 28 | 43 | 37 |

Après 4h de réaction, les catalyseurs acides zéolithiques présentent une activité plus faible qui s'accompagne d'une baisse de la sélectivité en diméthylisosorbide. La demanderesse a cependant cherché à remédier à ces inconvénients en augmentant le temps de séjour dans le lit catalytique (exemple 5) et/ou en utilisant des catalyseurs bifonctionnels métal-acide.

**Exemple 4 : O-méthylation de l'isosorbide par le méthanol catalysée par des catalyseurs solides acides non zéolithiques en réacteur continu en phase gazeuse à haute température.**

Les catalyseurs suivants sont évalués:

- Zircone tungstée: ZrW,
- Zircone sulfatée: ZrS,
- Zircone sulfétée dopée au Fe et au Mn : ZMFS

La réaction est mise en oeuvre dans le dispositif décrit dans l'exemple 2.

Le protocole expérimental suivant a été adopté :

- prélèvement du condensat au bout d'une heure et de 4h de réaction
- arrêt du pompage du mélange réactionnel au bout de quatre heures de réaction avec maintien du débit d'azote pendant 30 minutes.

Les conditions expérimentales sont les mêmes que celles de l'exemple 3.

Les résultats obtenus après 1h et 4h de réaction sont présentés dans les tableaux 5 et 6 respectivement.

**Tableau 5 :** O-méthylation de l'isosorbide par le méthanol catalysée par des catalyseurs solides acides ZrS, ZrW, ZMFS en réacteur continu en phase gazeuse. Résultats après 1h de réaction.

| Catalyseurs | Conversion Isosorbide (%) | Sélectivités (% molaire) | | |
|---|---|---|---|---|
| | | DMI | MMI B | MMI A |
| ZrS | 42 | 38 | 43 | 19 |
| ZMFS | 68 | 20 | 55 | 25 |
| ZrW | 63 | 37 | 42 | 21 |

Les catalyseurs acides à base de Zr catalysent l'éthérification de l'isosorbide par la MeOH en éthers méthyliques.

**Tableau 6 :** O-méthylation de l'isosorbide par le méthanol catalysée par des catalyseurs solides acides ZrS, ZrW, ZMFS en réacteur continu en phase gazeuse. Résultats après 4h de réaction.

| Catalyseurs | Conversion Isosorbide (%) | Sélectivités (% molaire) | | |
|---|---|---|---|---|
| | | DMI | MMI B | MMI A |
| ZrS | 25 | 13 | 52 | 35 |
| ZMFS | 9 | 0 | 47 | 53 |
| ZrW | 38 | 11 | 52 | 37 |

Comme dans le cas des catalyseurs zéolitiques, les catalyseurs acides à base de zircone semblent présenter une désactivation au cours de leur fonctionnement, celle-ci s'accompagne aussi d'une baisse de la sélectivité en diméthylisosorbide.

**Exemple 5 : O-méthylation de l'isosorbide par le méthanol catalysée par la zéolithe H-ZSM5 en réacteur continu en phase gazeuse à haute température. Recyclage des produits.**

Les produits d'éthérification de l'isosorbide par le méthanol obtenus à l'issu d'une réaction de 4h conduite dans les conditions de l'exemple 3 sont introduits dans le réacteur pour un nouveau cycle réactionnel dans des conditions identiques. Les conditions réactionnelles sont les mêmes que celles décrites dans l'exemple 3.

Les résultats obtenus sont présentés dans le tableau 7.

**Tableau 7 :** Composition du milieu réactionnel après un premier passage puis un second passage (recirculation des produits) sur le lit catalytique.

| | (% molaire) | | | |
|---|---|---|---|---|
| | MMI B | MMI A | DMI | isosorbide |
| $1^{er}$ passage | 25 | 13 | 6 | 56 |
| $2^{eme}$ passage | 32 | 14 | 19 | 35 |

La recirculation des produits de réaction sur le lit catalytique permet d'augmenter la conversion de l'isosorbide et, en particulier, la formation de DMI.

La conversion n'est donc limitée que par le temps de séjour dans le réacteur. Un premier perfectionnement à envisager consisterait à démultiplier le lit catalytique pour augmenter le temps de séjour des réactifs dans le lit catalytique et donc pour augmenter le taux de conversion. Industriellement, il s'agirait d'utiliser des colonnes avec un contenu en catalyseur plus important afin d'atteindre des taux de conversion plus élevés, en particulier proches du quantitatif.

**Exemple 6 : O-méthylation de l'isosorbide par le méthanol catalysée par H-ZSM5, ZrS et ZrW, évolution de l'activité catalytique au cours du temps**

Les conditions expérimentales sont les suivantes :

$m_{cata}$ = 2g
$P_{isosorbide}$ = 14 torr
$T_{vaporisation}$ = 185°C
$T_{réaction}$ = 200°C
Rapport molaire méthanol/isosorbide = 20
$ppH_{iso}$ ($h^{-1}$) = 0,39 g iso.g $cata^{-1}.h^{-1}$

La durée de la réaction est de 8h avec un prélèvement toutes les 2h.

**Tableau 8 :** Evolution de la conversion de l'isosorbide au cours du temps.

| Catalyseurs | Isosorbide conversion (%) | | | |
|---|---|---|---|---|
| | 2h | 4h | 6h | 8h |
| ZrS | 61 | 30 | 13 | 5 |
| ZSM5 | 53 | 30 | 21 | 17 |
| ZrW | 25 | 13 | 1 | 0 |

Quel que soit le catalyseur, l'activité décroît avec le temps de réaction. Cependant, ZSM5 présente une activité significativement plus élevée que ZrS et ZrW, Par ailleurs, l'activité de ZSM5 se stabilise autour de 20% de conversion d'isosorbide après 6h de réaction.

**Exemple 7 : O-méthylation de l'isosorbide par le méthanol catalysée par la un catalyseur bifonctionnel métal-acide : Pt dispersé sur H-ZSM5 en présence d'hydrogène**

Le catalyseur bifonctionnel est préparé par imprégnation à humidité naissante de 1% en poids de Pt sur la H-ZSM-5.

Les conditions réactionnelles sont les mêmes que celles de l'exemple 6.

Les résultats sont présentés sur le tableau 9.

**Tableau 9 :** O-méthylation de l'isosorbide par le méthanol catalysée par Pt/H-ZSM-5 en présence d'hydrogène.

| % mol | Isosorbide conversion (%) | | | |
|---|---|---|---|---|
| | 2h | 4h | 6h | 8h |
| conversion | 83 | 72 | 61 | 55 |
| Se DMI | 32 | 27 | 21 | 18 |
| Se MMI B | 59 | 54 | 50 | 49 |
| Se MMI A | 9 | 19 | 29 | 33 |

Par rapport au catalyseur monofonctionnel acide H-ZSM-5 (exemple 6), l'ajout de Pt au catalyseur H-ZSM-5, couplé à la présence d'$H_2$ dans le flux permet de limiter la vitesse de désactivation du catalyseur au cours du temps tout en limitant la baisse de la sélectivité en DMI. Il est probable par ailleurs qu'une optimisation de la balance fonction acide/fonction métallique du catalyseur, tout comme l'optimisation de la $pph_{iso}$, puisse permettre de limiter la désactivation et stabiliser l'activité.

**Exemple 8 : O-éthylation de l'isosorbide par l'éthanol en phase gazeuse catalysée par H-ZSM-5**

Les conditions réactionnelles sont les mêmes que celles de l'exemple 3. Le rapport molaire éthanol/isosorbide est de 20.

Les résultats sont présentés sur le tableau 10.

**Tableau 10 :** O-éthylation de l'isosorbide par l'éthanol catalysée par Pt/H-ZSM-5 en présence d'hydrogène

| Temps | Conversion Isosorbide (%) | Sélectivités (% molaire) | | |
|---|---|---|---|---|
| | | DEI | MEI B | MEI A |
| 0h-1h | 47 | 23 | 55 | 22 |
| 1h-4h | 40 | 15 | 57 | 28 |

La O-éthylation de l'isosorbide par l'éthanol est réalisable en réacteur continu en phase gazeuse à haute température en présence du catalyseur H-ZSM5. Les résultats obtenus au bout d'une heure montrent la formation de diéthylisosorbide (DEI) et de composés monoéthylés. Une baisse de l'activité et de la sélectivité en DEI parait au cours du temps. Toutefois, la désactivation est moins prononcée qu'en présence de MeOH.

## Revendications

1. Procédé de préparation d'une composition à base de dialkyloxydianhydrohexitols par éthérification de 1,4 :3,6-dianhydrohexitols avec au moins un agent d'O-alkylation, en présence d'un catalyseur solide, et **caractérisé en ce que** l'agent d'éthérification est choisi dans le groupe comprenant:

   - les alcools;
   - et leurs mélanges.

2. Procédé selon la revendication 1 **caractérisé en ce que** l'éthérification est au moins en partie effectuée en phase gazeuse.

3. Procédé selon la revendication 1 ou 2 **caractérisé en ce que** le catalyseur est choisi dans le groupe comprenant:

   - les sels des hétéropolyacides ou polyoxométallates de formule générale:

   $$H_kX_jM_mO_n, yH_2O \qquad (I)$$

   dans laquelle,

- X représente un hétéroatome choisi dans le groupe constitué par les éléments suivants : P, Si, Ge, B et As,
- M représente un élément métallique périphérique choisi dans le groupe constitué par W, Mo et V,
- j est le nombre d'hétéroatomes et représente 1 ou 2,
- k est le nombre d'atomes d'hydrogène et est compris entre 0,5 et 10,
- m est le nombre d'atomes métalliques périphériques W, Mo, V, et est compris entre 1 et 18,
- n est le nombre d'atomes d'oxygène et est compris entre 2 et 62,
- y est le nombre de molécules d'eau d'hydratation et est compris entre 0 et 40;
- et leurs mélanges;

- les sels de métaux alcalins $Cs^+$, $K^+$, $Rb^+$ et les sels d'ammonium $NH_4^+$ et leurs mélanges;
- les catalyseurs acides à base d'oxyde de zirconium modifié par des oxoanions de type sulfate ou tungstate d'appellation ZrS ou ZrW pouvant contenir des métaux de transition tels Fe, Mn et leurs mélanges;
- les zéolithes;
- les argiles acides de type montmorillonites, les phosphates, les charbons fonctionnalisés ; et leurs mélanges
- et leurs mélanges.

**4.** Procédé selon la revendication 3 **caractérisé en ce que** pour le catalyseur, les sels des hétéropolyacides de formule générale (I) sont choisis dans le groupe comprenant :

- $H_3PW_{12}O_{40}$,$21H_2O$ ;
- $H_4SiW_{12}O_{40}$,$24H_2O$ ;
- $H_6P_2W_{18}O_{62}$,$24H_2O$ ;
- $H_5BW_{12}O_{40}$,$30H_2O$ ;
- $H_5PW_{10}V_2O_{40}$,$yH_2O$ ;
- $H_3PMo_{12}O_{40}$,$28H_2O$ ;
- $H_4SiMo_{12}O_{40}$,$13H_2O$ ;
- $H_3PMo_6V_6O_{40}$,$yH_2O$ ;
- $H_5PMo_{10}V_2O_{40}$,$yH_2O$ ;
- et leurs mélanges.

**5.** Procédé selon l'une au moins des revendications précédentes **caractérisé en ce que** le catalyseur est supporté.

**6.** Procédé selon l'une au moins des revendications précédentes **caractérisé en ce que** le catalyseur comprend un métal noble,
et **en ce que** l'éthérification est au moins en partie effectuée sous flux d'hydrogène.

**7.** Procédé selon l'une au moins des revendications précédentes caractérisé en qu'il comprend une étape de régénération du catalyseur solide.

**8.** Procédé selon l'une au moins des revendications précédentes **caractérisé**

- **en ce que** les 1,4:3,6-dianhydrohexitols comprennent un dérivé de 1,4:3,6-dianhydrohexitol de formule II :

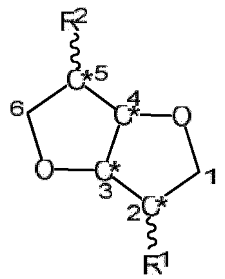

(II)

dans laquelle :

$R^1$, $R^2$ est un radical $-OR^3$, les radicaux $R^3$ étant identiques ou différents et correspondant chacun à H ou

un alkyle ;

- et **en ce que** les dialkyloxydianhydrohexitols comprennent un dérivé de 1,4:3,6-dianhydrohexitol de formule III :

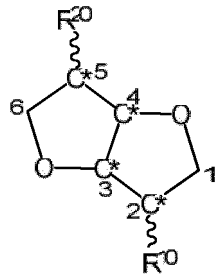

(III)

dans laquelle :

$R^{10}$, $R^{20}$ est un radical $-OR^{30}$, les radicaux $R^{30}$ étant identiques ou différents et correspondant chacun à un alkyle.

**9.** Procédé selon l'une au moins des revendications précédentes caractérisé en que le rapport molaire [agent d'alkylation / dianhydrohexitol] est inférieur ou égal à 30.

**10.** Procédé selon l'une au moins des revendications précédentes caractérisé en que :

1. dans une première étape, on vaporise le(s) 1,4:3,6-dianhydrohexitol(s) à une température de T1 (en °C) supérieure ou égale à 170;
2. dans une deuxième étape, on effectue l'éthérification avec l'agent d'alkylation à une température T2 (en °C) supérieure ou égale à T1.

**11.** Procédé selon l'une au moins des revendications précédentes **caractérisé en ce que** le(s) 1,4 :3,6-dianhydrohexitol(s) de départ est fondu, en solution et/ou provient directement de la synthèse de 1,4 :3,6-dianhydrohexitol(s) à partir d'hexitol(s).

**12.** Procédé selon l'une au moins des revendications précédentes **caractérisé en ce que** l'on met en oeuvre, à titre de produit(s) de départ, un (des) hexitol(s) et **en ce que** la déshydratation de l'hexitol est catalysée par le catalyseur dans une étape unique intégrant la déshydratation de l'(des) hexitol(s) en 1,4 :3,6-dianhydrohexitol(s) l'éthérification du(des)dit(s) 1,4 :3,6-dianhydrohexitol(s).

**13.** Procédé selon l'une au moins des revendications précédentes caractérisé en qu'il est mis en oeuvre selon un mode continu ou semi-continu.

## Patentansprüche

**1.** Verfahren zur Herstellung einer Zusammensetzung auf Basis von Dialkyloxydianhydrohexitolen durch Veretherung von 1,4:3,6-Dianhydrohexitolen mit mindestens einem O-Alkylierungsmittel in Gegenwart eines festen Katalysators, und **dadurch gekennzeichnet, dass** das Veretherungsmittel aus der Gruppe ausgewählt ist umfassend:

- Alkohole;
- und ihre Gemische.

**2.** Verfahren nach Anspruch 1 **dadurch gekennzeichnet, dass** die Veretherung mindestens zum Teil in der Gasphase durchgeführt wird.

**3.** Verfahren nach Anspruch 1 oder 2, **dadurch gekennzeichnet, dass** der Katalysator aus der Gruppe ausgewählt ist umfassend:

- Salze der Heteropolysäuren oder Polyoxometallate der allgemeinen Formel:

$$H_kX_jM_mO_n, yH_2O \qquad (I)$$

wobei

- X ein Heteroatom darstellt, das aus der Gruppe ausgewählt ist bestehend aus den folgenden Elementen: P, Si, Ge, B und As,
- M ein peripheres metallisches Element darstellt, das aus der Gruppe ausgewählt ist bestehend aus W, Mo und V,
- j die Anzahl von Heteroatomen ist und 1 oder 2 bedeutet,
- k die Anzahl von Wasserstoffatomen ist und von 0,5 bis 10 beträgt,
- m die Anzahl von peripheren Metallatomen W, Mo, V ist und von 1 bis 18 beträgt,
- n die Anzahl von Sauerstoffatomen ist und von 2 bis 62 beträgt,
- y die Anzahl von Hydratwassermolekülen ist und von 0 bis 40 beträgt;
- und ihre Gemische;

- Alkalimetallsalze $Cs^+$, $K^+$, $Rb^+$ und Ammoniumsalze $NH_4^+$ und ihre Gemische;
- Säure-Katalysatoren auf Basis von Zirkoniumoxid, das durch Oxoanionen vom Typ Sulfat oder Wolframat der Bezeichnung ZrS oder ZrW modifiziert ist, die Übergangsmetalle wie Fe, Mn und ihre Gemische enthalten können;
- Zeolithe;
- saure Tone vom Typ Montmorillonite, Phosphate, funktionalisierte Kohlenstoffe; und ihre Gemische.
- und ihre Gemische.

4. Verfahren nach Anspruch 3 **dadurch gekennzeichnet, dass** für den Katalysator die Salze der Heteropolysäuren der allgemeinen Formel (I) aus der Gruppe ausgewählt sind umfassend:

- $H_3PW_{12}O_{40},21H_2O$ ;
- $H_4SiW_{12}O_{40},24H_2O$ ;
- $H_6P_2W_{18}O_{62},24H_2O$ ;
- $H_5BW_{12}O_{40},30H_2O$ ;
- $H_5PW_{10}V_2O_{40},yH_2O$ ;
- $H_3PMo_{12}O_{40},28H_2O$ ;
- $H_4SiMo_{12}O_{40}, 13H_2O$ ;
- $H_3PMo_6V_6O_{40},yH_2O$ ;
- $H_5PMo_{10}V_2O_{40},yH_2O$ ;
- und ihre Gemische.

5. Verfahren nach mindestens einem der vorangehenden Ansprüche, **dadurch gekennzeichnet, dass** der Katalysator trägergebunden ist.

6. Verfahren nach mindestens einem der vorangehenden Ansprüche, **dadurch gekennzeichnet, dass** der Katalysator ein Edelmetall umfasst, und dadurch, dass die Veretherung mindestens zum Teil unter Wasserstrom durchgeführt wird.

7. Verfahren nach mindestens einem der vorangehenden Ansprüche, **dadurch gekennzeichnet, dass** es einen Regenerierungsschritt des festen Katalysators umfasst.

8. Verfahren nach mindestens einem der vorangehenden Ansprüche,

- **dadurch gekennzeichnet, dass** die 1,4:3,6-Dianhydrohexitole ein Derivat von 1,4:3,6-Dianhydrohexitol der Formel II umfassen:

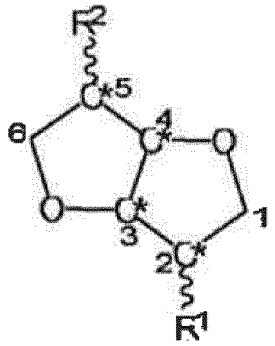

(II)

wobei

$R^1$, $R^2$ ein Radikal -$OR^3$ ist, wobei die Radikale $R^3$ gleich oder verschieden sind und jeweils H oder einem Alkyl entsprechen;

- und dadurch, dass die Dialkyloxydianhydrohexitole ein Derivat von 1,4:3,6-Dianhydrohexitol der Formel III umfassen:

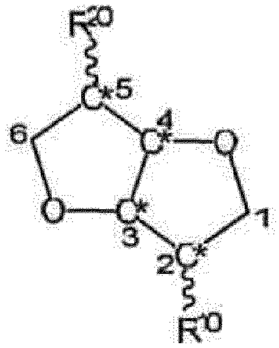

(III)

wobei

$R^{10}$, $R^{20}$ ein Radikal -$OR^{30}$ ist, wobei die Radikale $R^{30}$ gleich oder verschieden sind und jeweils einem Alkyl entsprechen.

**9.** Verfahren nach mindestens einem der vorangehenden Ansprüche, **dadurch gekennzeichnet, dass** das Molverhältnis [Alkylierungsmittel / Dianhydrohexitol] kleiner oder gleich 30 ist.

**10.** Verfahren nach mindestens einem der vorangehenden Ansprüche, **dadurch gekennzeichnet, dass**:

1. in einem ersten Schritt, das (die) 1,4:3,6-Dianhydrohexitol(e) bei einer Temperatur T1 (in °C) von größer oder gleich 170 verdampft werden;
2. in einem zweiten Schritt die Veretherung mit dem Alkylierungsmittel bei einer Temperatur T2 (in °C) von größer oder gleich T1 durchgeführt wird.

**11.** Verfahren nach mindestens einem der vorangehenden Ansprüche, **dadurch gekennzeichnet, dass** das (die) Ausgangs-1,4:3,6-Dianhydrohexitol(e) geschmolzen ist, in Lösung ist und/oder direkt aus der Synthese von 1,4:3,6-Dianhydrohexitol(n) aus Hexitol(en) stammt.

**12.** Verfahren nach mindestens einem der vorangehenden Ansprüche, **dadurch gekennzeichnet, dass** als Ausgangsprodukt(e) (ein) Hexitol(e) eingesetzt wird (werden) und dadurch, dass die Dehydratation des Hexitols durch den Katalysator in einem einzigen Schritt katalysiert wird, der die Dehydratation des (der) Hexitols(e) 1,4:3,6-Dianhydrohexitol(en) die Veretherung des (der) 1,4:3,6-Dianhydrohexitol(e) integriert.

13. Verfahren nach mindestens einem der vorangehenden Ansprüche, **dadurch gekennzeichnet, dass** es kontinuierlich oder halbkontinuierlich durchgeführt wird.

## Claims

1. Method for preparing a composition based on dialkyloxydianhydrohexitols by etherification of 1,4:3,6-dianhydrohexitols with at least one O-alkylating agent, in the presence of a solid catalyst, and **characterized in that** the etherification agent is selected from the group comprising:

    - alcohols,
    - and mixtures thereof.

2. Method according to claim 1, **characterized in that** the etherification is at least partly effected in gas phase.

3. Method according to claim 1 or 2, **characterized in that** the catalyst is selected from the group comprising:

    - salts of heteropolyacids or polyoxometallates of general formula:

    $$H_kX_jM_mO_n, yH_2O \qquad (I)$$

    wherein,

    - X represents a heteroatom selected from the group consisting of the following elements: P, Si, Ge, B and As,
    - M represents a peripheral metallic element selected from the group consisting of W, Mo and V,
    - j is the number of heteroatoms and represents 1 or 2,
    - k is the number of hydrogen atoms and is between 0.5 and 10,
    - m is the number of peripheral metal atoms W, Mo and V and is between 1 and 18,
    - n is the number of oxygen atoms and is between 2 and 62,
    - y is the number of molecules of water of hydration and is between 0 and 40,
    - and mixtures thereof,

    - salts of alkali metals $Cs^+$, $K^+$, $Rb^+$ and ammonium $NH_4^+$ salts and mixtures thereof,
    - acidic catalysts based on zirconium oxide modified with oxo anions of the sulfate or tungstate type referred to as ZrS or ZrW possibly containing transition metals such as Fe, Mn and mixtures thereof,
    - zeolites,
    - acidic clays of the montmorillonite type, phosphates, functionalized carbons, and mixtures thereof,
    - and mixtures thereof.

4. Method according to claim 3, **characterized in that** for the catalyst, the salts of the heteropolyacids of general formula (I) are selected from the group comprising:

    - $H_3PW_{12}O_{40}\cdot 21H_2O$,
    - $H_4SiW_{12}O_{40}\cdot 24H_2O$,
    - $H_6P_2W_{18}O_{62}\cdot 24H_2O$,
    - $H_5BW_{12}O_{40}\cdot 30H_2O$,
    - $H_5PW_{10}V_2O_{40}\cdot yH_2O$,
    - $H_3PMo_{12}O_{40}\cdot 28H_2O$,
    - $H_4SiMo_{12}O_{40}\cdot 13H_2O$,
    - $H_3PMo_6V_6O_{40}\cdot yH_2O$,
    - $H_5PMo_{10}V_2O_{40}\cdot yH_2O$,
    - and mixtures thereof.

5. Method according to at least one of the previous claims, **characterized in that** the catalyst is supported.

6. Method according to at least one of the previous claims, **characterized in that** the catalyst comprises a noble metal, and **in that** the etherification is at least partly effected under a stream of hydrogen.

7. Method according to at least one of the previous claims, **characterized in that** it includes a solid catalyst regeneration stage.

8. Method according to at least one of the previous claims, **characterized**

- **in that** the 1,4:3,6-dianhydrohexitols comprise a derivative of 1,4:3,6-dianhydrohexitol of formula II:

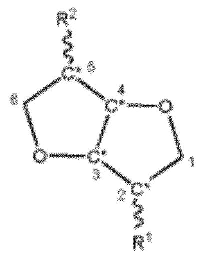

(II)

wherein:

$R^1$ and $R^2$ is an $-OR^3$ radical, the radicals $R^3$ being identical or different and each corresponding to H or an alkyl,

- and **in that** the dialkyloxydianhydrohexitols comprise a derivative of 1,4:3,6-dianhydrohexitol of formula III:

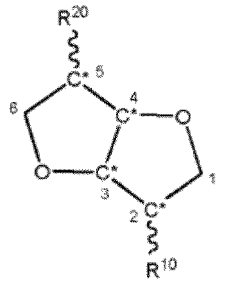

(III)

wherein:

$R^{10}$ and $R^{20}$ is an $-OR^{30}$ radical, the radicals $R^{30}$ being identical or different and each corresponding to an alkyl.

9. Method according to at least one of the previous claims, **characterized in that** the [alkylating agent/ dianhydrohexitol] molar ratio is less than or equal to 30.

10. Method according to at least one of the previous claims, **characterized in that**:

1. in a first stage, the dianhydrohexitol(s) is/are vaporized at a temperature of T1 (in °C) greater than or equal to 170
2. in a second stage, the etherification is effected with the alkylating agent at a temperature T2 (in °C) greater than or equal to T1.

11. Method according to at least one of the previous claims, **characterized in that** the starting 1,4:3,6-dianhydrohexitol(s) is/are melted, in solution and/or derive(s) directly from the synthesis of 1,4:3,6-dianhydrohexitol(s) from hexitol(s).

12. Method according to at least one of the previous claims, **characterized in that**, as the starting product(s), hexitol(s) is/are used, and **in that** the dehydration of the hexitol is catalyzed by the catalyst in a single stage combining the dehydration of the hexitol(s) to 1,4:3,6-dianhydrohexitol(s) and the etherification of the 1,4:3,6-dianhydrohexitol(s).

**13.** Method according to at least one of the previous claims, **characterized in that** it is implemented according to a continuous or semi-continuous mode.

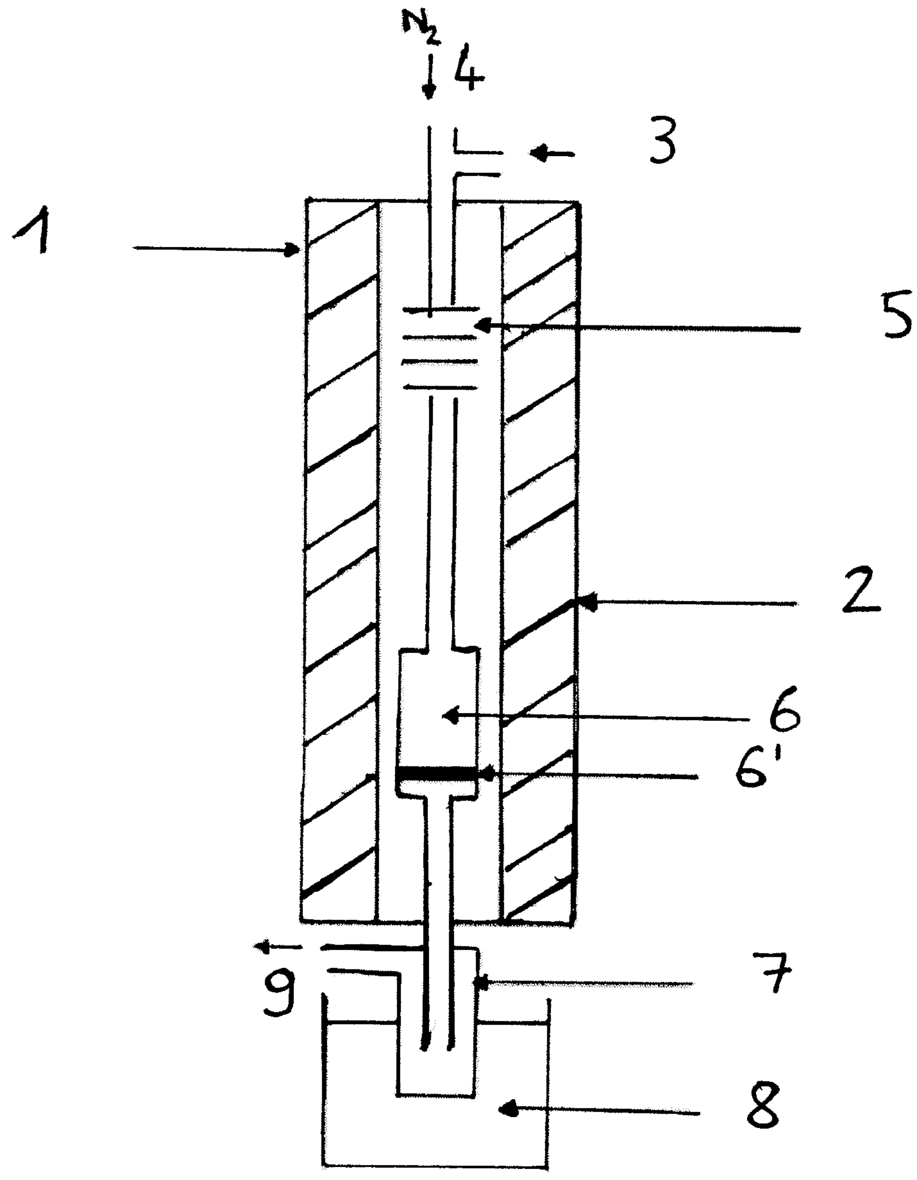


FIG. 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2006120342 A
- WO 2006120343 A
- EP 0092998 A
- US 4770871 A
- WO 2009120703 A


### Littérature non-brevet citée dans la description


- Les techniques physiques d'étude des catalyseurs. 1988